(19) Europäisches Patentamt
European Patent Office
Office européen des brevets

(11) Publication number: **0 138 530 A2**

# (12) EUROPEAN PATENT APPLICATION

(21) Application number: **84306810.7**

(22) Date of filing: **05.10.84**

(51) Int. Cl.⁴: **C 12 Q 1/00**

(30) Priority: **05.10.83 GB 8326696**

(43) Date of publication of application: **24.04.85**
**Bulletin 85/17**

(84) Designated Contracting States: **AT BE CH DE FR GB IT LI NL SE**

(71) Applicant: **The Secretary of State for Social Services in Her Britannic Majesty's Government of the United Kingdom of Great Britain and, Northern Ireland 14 Russell Square, London WC1B 5EP (GB)**
Applicant: **The Public Health Laboratory Service Board, 61 Colindale Avenue, London NW9 5EQ (GB)**

(72) Inventor: **Atkinson,Anthony, Twingley Mill Corner Winterbourne Gunner, Salisbury Wiltshire (GB)**
Inventor: **Scawen, Michael Denis, 14 Paddock Close Winterbourne Dauntsey, Salisbury Wiltshire (GB)**
Inventor: **Campbell, Robert Stewart, 14 Lacey's Lane Exning, Newmarket Suffolk CB8 7HL (GB)**
Inventor: **Hammond, Peter Michael, 64 Eastfield Avenue Melton Mowbray, Leicestershire (GB)**
Inventor: **Price, Christopher Philip, Church End Mingle Lane Stapleford, Cambridge Cambridgeshire (GB)**

(74) Representative: **James, Stephen Richard et al, Procurement Executive Ministry of Defence Patents 1A(4), Room 2014 Empress State Building, Lillie Road London SW6 1TR (GB)**

## (54) Method for the estimation of salicylates or reduced Pyridine nucleotides.

(57) A method for the estimation of a salicylate or a reduced pyridine nucleotide (especially NADH or NAD(P)H) in a sample consisting of enzymically converting a salicylate to a catechol by the action of a salicylate mono-oxygenase enzyme on the salicylate in the presence of a reduced pyridine nucleotide, reacting the catechol with an aminophenol, preferably para-aminophenol, to form an indophenol and estimating the quantity of the indophenol spectrophotometrically, the quantity of the indophenol being a measure of the salicylate or reduced pyridine nucleotide in the sample.

A diagnostic kit to allow the routine use of the method is also provided.

EP 0 138 530 A2

Europe

0138530

JX/6506



# METHOD FOR THE ESTIMATION OF SALICYLATES OR REDUCED PYRIDINE NUCLEOTIDES

This invention relates to a method for the estimation of salicylates or reduced pyridine nucleotides, and to a diagnostic kit adapted to facilitate the routine performance of said method.

A number of methods for estimating salicylate in a sample or in a solution are known. For example, the salicylate ion can be reacted with the Folin-Ciocalteau reagent in a strongly alkaline solution to produce a blue colour which is then analysed colorimetrically (Smith & Talbot 1950, Brit J Exp Path. 31, 65). This method, however, gives high and variable blank values in samples where salicylates are absent.

Alternatively, the salicylate ion can be reacted with ferric salts in acidic solution and the purple colour produced analysed by a colorimetric means. (Trinder 1954, Biochem J. 57, 301). This method too has disadvantages, and is subject to non-specific interference.

In another method of salicylate estimation, the salicylate is first converted enzymically to form pyrocatechol. The latter compound is then reacted with hydrazone and molecular oxygen in the presence of a second enzyme, tyrosinase, to form an azine dye. (Roder et al 1980, EP 0 054 146). The enzyme tyrosinase is known to oxidise monophenols other than pyrocatechol, and hydrazone may couple oxidatively with any resulting quinones to form a coloured product. Furthermore, the presence of other monophenols and particularly of tyrosine in unknown quantities may result in competition for binding sites on the tyrosinase enzyme, reducing the efficiency of the system.

There is a requirement to estimate the level of salicylate compounds, particularly the drugs ortho-acetyl salicylic acid (aspirin) and salicylic acid (which is commonly used as its sodium salt) in a biological fluid. In such cases, and especially where a patient is suffering from a drug overdose, it is

important that the drug level in the body may be assessed rapidly so that an antidote or other treatment may, if necessary, be administered. Although the above described methods are useful in meeting this requirement to some extent, they have disadvantages in that they may require solvent extraction or suffer from interfering activity.

It is one object of the present invention to provide a method for the estimation of salicylate by which the above disadvantages are overcome or at least mitigated, but which still produces an estimation of salicylate level sufficiently quickly to allow the method to be used in the determination of drug levels in the biological fluids of suspected overdose patients.

According to a first aspect of the present invention, therefore, there is provided a method for the estimation of a salicylate in a sample comprising enzymically converting the salicylate to a catechol by the action of a salicylate mono-oxygenase enzyme on the salicylate in the presence of a reduced pyridine nucleotide (especially NADH or NAD(P)H), reacting the catechol with an aminophenol to form an indophenol and estimating the quantity of the indophenol spectrophotometrically, the quantity of the indophenol being a measure of the salicylate in the sample. Preferably the indophenol is estimated colormetrically.

The invention is based on the following reactions (using NAD(P)H or NADH to exemplify the reduced pyridine nucleotide)

(i) Salicylate + (NAD(P)H) (or NADH) + $O_2$ $\xrightarrow[\text{EC: 1.14.13.1}]{\text{Salicylate Mono-oxygenase}}$ Catechol + $CO_2$ + $H_2O$ + (NAD(P)) (or NAD) ...(A)

(ii) Catechol + Aminophenol $\longrightarrow$ Indophenol ...(B)

In reaction A above, the reduced pyridine nucleotide comprising either reduced nicotinamide adenine dinucleotide (NADH) or reduced nicotinamide adenine dinucleotide phosphate (NAD(P)H)

is converted to its respective oxidised form nicotinamide adenine dinucleotide (NAD) or nicotinamide adenine dinucleotide phosphate (NAD(P)).

The conversion of the salicylate to the catechol is catalysed by any salicylate mono-oxygenase (decarboxylating and hydroxylating) by the International Union of Biochemistry (Enzyme Nomenclature 1978, Academic Press, New York, 1979). In thecatalysed reaction the enzyme and the reduced pyridine nucleotide are present in excess. If the assay reaction mixture contains high concentrations of metal ions then a metal chelating agent, preferably in the concentration 0.1 to 1.0 mmol should also be added to safeguard enzyme activity. Where the salicylate is present in the form of an acetylated derivative, it should first be converted to its non-acetylated form by hydrolysis of the acetylated derivative for example in a reaction catalysed by any enzyme of the type defined as EC: 3.1.1.2. and named as arylester hydrolase by the International Union of Biochemistry (reference as above). The unsubstituted form of the present indophenol has the Formula I

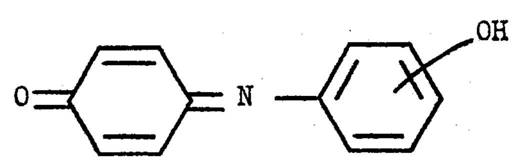

The conversion of catechol to indophenol is performed by reacting the catechol with an amino substituted phenol, especially *para*-aminophenol. In this especially preferred embodiment the reaction of catechol (with *para*-aminophenol) is fast, highly specific, and produces a relatively colour-stable dye. Preferably, the conversion of catechol to indophenol is performed in a substantially aqueous solution, at a neutral or, which is preferred, an alkaline pH. In a particularly preferred embodiment of the present invention the conversion is effected at a pH of

above 10.0, especially about pH 12. to 13. When the formation of the indophenol is carried out under conditions appropriate to allow a maximum rate of colour formation, it may be completed in under three minutes with serum samples.

Once produced, the dye is generally stable for at least one hour. The colour-producing reaction with aminophenol, especially para-aminophenol, is highly specific. No interfering colour formation is observed in the reaction of aminophenol with salicylate, acetylated salicylate, acylanilides, such as hydroxyacetanilide, or with tyrosine. The present procedure, therefore provides a rapid and specific method for the determination of salicylate which will be particularly useful when the salicylate is present in biological fluids.

The spectrophotometric analysis of the indophenol may be carried out at any wavelength at which the dye absorbs, but is preferably carried at the wavelength of maximum absorption. When the indophenol is formed by reaction of catechol with para-aminophenol this wavelength is 565 nm.

The present method of detecting salicylates will have many applications, but will be of particular use when a rapid but routine method of analysis is required, especially when the fluid in which the salicylate is present contains other constituents which are known to interfere in alternative methods of analysis. The present method will be particularly useful in the medical field for the detection of analgesic formulations based upon the drug salicylate. In this case the salicylate will tend to be in admixture with other drug derivatives that are known to interfere in the traditional means of rapid analysis.

The requirement for a co-factor, especially either NAD(P)H or NADH, in the enzymatic conversion of salicylate to catechol by salicylate mono-oxygenase, means that the present reaction scheme may also be employed in the estimation of a reduced pyridinenucleotide, especially either NADH or NAD(P)H. Examples of reactions where NADH or HAD(P)H may be produced (and then

estimated) include the action of dehydrogenase enzymes on substrates such as glucose or triglycerides, or lactate .. Accordingly, the present invention further provides in a second aspect of the present invention a method for the estimation of a reduced pyridine nucleotide (especially NADH or NAD(P)H) in a sample comprising enzymically converting a salicylate to a catechol by the action of a salicylate mono-oxygenase enzyme on the salicylate in the presence of the reduced pyridine nucleotide, reacting the catechol with an aminophenol to form an indophenol, and estimating the quantity of the indophenol spectrophotometrically, the quantity of the indophenol being a measure of the reduced pyridine nucleotide in the sample. Preferably the indophenol is estimated colorimetrically.

The method for estimating a reduced pyridinenucleotide is also based on reactions (A) and (B) above. However, in this case, unlike the estimation of salicylate (when enzyme and reduced pyridine nucleotide are in excess), the salicylate and the enzyme are present in excess. As before, the catechol is conveniently converted to an indophenol by aminophenol.

In order to further facilitate the use of the present methods of analysis in a routine manner, especially in medical techniques, the invention further provides a diagnostic kit for use in the estimation of a salicylate, or a reduced pyridine nucleotide (especially NADH or NAD(P)H) by a process of the present invention comprising:

a. a salicylate mono-oxygenase enzyme suitable for the conversion of salicylate to a catechol in the presence of a reduced pyridinenucleotide,

b. an amino phenol suitable for the conversion of the catechol to an indophenol.

As outlined above, the salicylate mono-oxygenase enzyme is any of those defined as EC: 1.14.13.1 by the International Union of Biochemistry. The aminophenol is preferably p-aminophenol.

The enzyme may be in solution, preferably aqueous solution. In this case, in order to retain the activity of the salicylate mono-oxygenase enzyme over prolonged periods of time, the enzyme solution preferably contains glycerol, or may be lyophilised in the presence of a protective agent. This protective agent may be a sugar, most especially glucose, lactose, raffinose or a polysaccharide.

Alternatively, the enzyme may be impregnated onto a solid or a semi-solid support. In this case a porous material, such as paper, is particularly preferred.

In order to further retain the activity of the enzyme, it may be stored at low temperature ($5^{o}$ to $-20^{o}C$) either with the other components of the kit or separately from them.

The aminophenol may also be present in the kit in the form of a solution, preferably an aqueous solution. Alternatively the aminophenol may be impregnated onto a solid or a semi-solid support. In this case a porous material, such as paper, is particularly preferred.

In one embodiment of the present kit both the enzyme and the aminophenol are inpregnated onto a porous material, such as paper, which is then used in the form of a test strip.

In order to facilitate the use of the kit of this invention, each kit may be supplied with a set of instructions setting out the steps involved in the assay procedure.

The application of the present process is further illustrated by the following examples.

<u>Example 1: Salicylate estimation</u>

<u>Reagents</u> (all in aqueous solution)

| | | |
|---|---|---|
| Salicylate mono-oxygenase | 1.0 U $ml^{-1}$ | ) as one reagent |
| NADH or NAD(P)H | 0.35 mmol $l^{-1}$ | ) |
| Tris hydrochloride pH 8.2 | 100 mmol $l^{-1}$ | ) |
| p-Aminophenol (in 0.1 mol $litre^{-1}$ HCL) | 0.9 mmol $l^{-1}$ | |
| Sodium hydroxide | 0.25 mol $l^{-1}$ | |

Procedure

50 µl of sample was mixed with 1000 µl of buffer NADH (or NAD(P)H) salicylate mono-oxygenase solution and incubated at room temperature for 5 minutes. The sample solution was a standard aqueous solution containing a known molar concentration of salicylate ions. To the above reaction mixture were added 1.0 ml sodium hydroxide solution and 1.0 ml para-aminophenol solution and the mixture was retained at room temperature for 3 minutes. The absorbance at 565 nm of the dye produced in this mixture was then read, and the absorbance value plotted on a graph against the known salicylate molar concentration in the original sample.

The above procedure was repeated for several further standard sample solutions each containing different but known concentrations of salicylate ions up to a maximum salicylate ion concentration of 6 mmol $l^{-1}$, so that a standard calibration curve of salicylate molar concentration in solution against absorbance was built up. This curve indicated that the assay was linear up to a salicylate concentration of 5.0 mmol $l^{-1}$ in the original sample solution.

50 µl of an aqueous sample solution containing an unknown concentration of salicylate ions was subjected to the above reaction procedure, and its salicylate concentration was then read from the standard salicylate calibration curve.

Using the above procedure, accurate estimations of salicylate concentrations up to 5.0 mmol $l^{-1}$ in sample solutions could be made. For sample solutions containing higher concentrations, dilution of the sample stock solutions with known volumes of water was first required before measuring out the sample solution volume of 50 µl.

Example 2: Reduced pyridine nucleotide (NADH) estimation

Reagents (all in aqu eous solution)

| | |
|---|---|
| Salicylate mono-oxygenase | 20 U $ml^{-1}$ |
| Sodium salicylate | 100 mmol $l^{-1}$ |
| Tris hydrochloride, pH 8.2 | 100 mmol $l^{-1}$ |
| p-Aminophenol (in 0.1 mol litre $^{-1}$ HCL) | 0.9 mmol $l^{-1}$ |
| Sodium hydroxide | 0.25 mol $l^{-1}$ |

Procedure

750 µl Tris hydrochloride buffer solution, 100 µl sodium salicylate solution, and 100 µl salicylate mono-oxygenase solution were mixed with 50 µl sample solution and incubated at 25°C for 5 minutes. The sample solution was a standard aqueous solution containing a known molar concentration of NADH. To the above reaction mixture, 1.0 ml sodium hydroxide solution and 1.0 ml para-aminophenol solution were added with mixing and the solution was retained at room temperature for 3 minutes. The absorbance at 565 nm of the dye produced in this mixture was then read, and the absorbance value plotted on a graph against the known NADH concentration in the original sample.

The above procedure was repeated for several further standard sample solutions each containing known but different concentrations of NADH up to a maximum NADH concentration of 6 mmol $l^{-1}$ so that a calibration curve of NADH concentration in solution against absorbance was built up. This curve indicated that the assay was linear up to a NADH concentration of 5.0 mmol $l^{-1}$ in the original sample solution.

50 µl of an aqueous sample solution containing an unknown concentration of NADH was then subjected to the above reaction procedure and its NADH concentration then read from the standard NADH calibration curve.

Using the above procedure, accurate estimations of NADH concentrations up to 5.0 mmol $l^{-1}$ in sample solutions could be made. For samples containing higher concentrations, dilution of the sample stock solutions with known volumes of water was first required before measuring out the sample volume of 50 µl.

Example 3: Reduced pyridine nucleotide (NAD(P)H) estimation

Using identical reagents to those of Example 2 above, the procedure of Example 2 was used to build up a standard calibration curve of NAD(P)H concentration in solution against absorbance (linear to 5.0 mmol $l^{-1}$ NAD(P)H) using standard aqueous sample solutions each containing known but different concentrations of NAD(P)H rather than NADH.

50 µl of an aqueous sample solution containing an unknown concentration of NAD(P)H was then subjected to the above reaction procedure and its NAD(P)H concentration then read from the standard NAD(P)H calibration curve.

Example 4: Reduced pyridine nucleotide (NADH) estimation

Reagents (all in aqueous solution)

| | |
|---|---|
| Salicylate mono-oxygenase | 20 U $ml^{-1}$ |
| Sodium salicylate | 100 $mmol^{-1}$ |
| Tris hydrochloride, pH 8.2 | 150 mmol $l^{-1}$ |
| NAD | 10 mmol $l^{-1}$ |
| Lactate dehydrogenase (EC 1.1.1.27) | 10 U $ml^{-1}$ |
| p-Aminophenol (in 0.1 mol litre $^{-1}$ HCl) | 0.9 mmol $l^{-1}$ |
| Sodium hydroxide | 0.25 mol $l^{-1}$ |

Procedure

The following procedure was used to estimate lactate concentration in a sample solution via the estimation of NADH.

550 µl Tris hydrochloride buffer solution, 100 µl salicylate mono-oxygenase solution, 100 µl sodium salicylate solution, 100 µl lactate dehydrogenase solution and 100 µl NAD solution were mixed with 50 µl sample solution and incubated at 25°C for 10 minutes. The sample solution was a standard aqueous solution containing a known molar concentration of lactate ions. In the foregoing reaction mixture, the NAD was converted to NADH by reaction with the lactate ions in the presence of the lactate dehydrogenase enzyme which acted as a catalyst, and the NADH so produced thus enabled the enzymic conversion of the salicylate ions to catechol to take place in the presence of the salicylate

mono-oxygenase enzyme. To the above reaction mixture, 1.0 ml sodium hydroxide solution and 1.0 ml *para*-aminophenol solution were added with mixing and the solution was allowed to stand at room temperature for 3 minutes. The absorbance at 565 nm of the dye produced in the mixture was then read, and this absorbance value plotted on a graph against the known lactate molar concentration in the original sample.

The above procedure was repeated for several further standard sample solutions each containing different but known concentrations of lactate ions up to a maximum lactate ion concentration of 6 mmol $l^{-1}$, so that a standard calibration curve of lactate ion molar concentration in solution against absorbance was built up. This curve indicated that the assay was linear up to a lactate concentration of 3.2 mmol $l^{-1}$ in the original sample solution.

50 µl of an aqueous solution containing an unknown concentration of lactate ions was subjected to the above reaction procedure, and its lactate ion concentrate then read from the standard lactate calibration curve.

**Example 5: Reduced pyridine nucleotide (NADH) estimation**

Reagents (all in aqueous solution)

| | |
|---|---|
| Salicylate mono-oxygenase | 20 U $ml^{-1}$ |
| Sodium salicylate | 50 mmol $l^{-1}$ |
| Tris hydrochloride pH 8.2 containing 20 mmol $l^{-1}$ $MgCl_2$ | 150 mmol $l^{-1}$ |
| ATP containing 10 U$ml^{-1}$ hexokinase (E.C.2.7.1.1) | 100 mmol $l^{-1}$ |
| NADP | 10 mmol $l^{-1}$ |
| Glucose-6-phosphate dehydrogenase (EC 1.1.1.49) | 10 U $ml^{-1}$ |
| *p*-Aminophenol (in 0.1 mol litre $^{-1}$ HCL) | 0.9 mmol $l^{-1}$ |
| Sodium hydroxide | 0.25 mol $l^{-1}$ |

Procedure

The following procedure was used to measure glucose concentration in a sample solution via the estimation of NAD(P)H.

50 µl samples of aqueous solutions containing known but different concentrations of glucose were mixed with 450 µl Tris hydrochloride buffer solution, 100 µl ATP solution containing hexokinase, 100 µl (NAD(P) solution, 100 µl glucose-6-phosphate dehydrogenase solution, 100 µl sodium salicylate solution and 100 µl salicylate mono-oxygenase solution. These mixtures were incubated at 25°C for 10 minutes. In these reaction mixtures, glucose was converted to glucose-6-phosphate, which reduced NAD(P) to NAD(P)H, and the NAD(P)H so produced enabled the enzymic conversion of salicylate to catechol to occur. To these reaction mixtures, 1.0 ml sodium hydroxide solution and 1.0 ml para-aminophenol solution were added, with mixing, and the solutions allowed to stand at room temperature for three minutes. The absorbances at 565 nm of the dye produced in the mixtures were then read, and these absorbance values plotted on a graph against the known glucose concentrations in the original samples. A standard calibration curve of glucose molar concentration against absorbance was thereby built up. The curve indicated that the assay was linear up to a glucose concentration of 5.0 mmol $l^{-1}$ in the original sample.

An aqueous solution containing an unknown concentration of glucose was subjected to the above reaction procedure, and its glucose molar concentration then read from the standard glucose calibration curve.

Example 6: Salicylate estimation

Reagents (all in aqueous solution)

| | |
|---|---|
| Aryl ester hydrolase (EC 3.1.1.2) | 20 U $ml^{-1}$ |
| Salicylate mono-oxygenase | 20 U $ml^{-1}$ |
| Tris hydrochloride pH 8.2 | 100 mmol $l^{-1}$ |
| NADH or NAD(P)H | 10 mmol $l^{-1}$ |
| p-Aminophenol (in 0.1 mol $litre^{-1}$ HCL) | 0.9 mmol $l^{-1}$ |
| Sodium hydroxide | 0.25 mol $l^{-1}$ |

Procedure

The following procedure was used to estimate acetyl salicylate

ion concentration in a sample solution via the estimation of salicylate ions.

650 μl potassium phosphate solution, 100 μl NADH or NAD(P)H solution, 100 μl aryl ester hydrolase solution and 100 μl salicylate mono-oxygenase solution were mixed with 50 μl sample solution and incubated at 25°C for 5 minutes. The sample solution was a standard aqueous solution containing a known molar concentration of acetyl salicylate ions. In the above reaction mixture, the aryl ester hydrolase enzyme first catalysed the conversion of all the acetyl salicylate ions to salicylate ions, and then the reduced pyridine nucleotide present in excess converted all the salicylate ions so produced to catechol in the presence of salicylate mono-oxygenase. To the above reaction mixture, 1.0 ml sodium hydroxide solution and 1.0 ml <u>para</u>- aminophenol solution were added with mixing and the solution was retained at room temperature for 3 minutes. The absorbance at 565 nm of the dye produced in the mixture was then read, and this absorbance value plotted on a graph against the known acetyl salicylate ion molar concentration in the original sample solution.

The above procedure was repeated for several further standard solutions, each containing different but known concentration of acetyl salicylate ions, up to a maximum acetyl salicylate ion concentration of 6 mmol $l^{-1}$, so that a standard calibration curve of acetyl salicylate ion molar concentration in solution against absorbance could be built up. This curve indicated that the assay was linear up to an acetyl salicylate ion concentration of 5.0 mmol $l^{-1}$ in the original sample solution.

50 μl of an aqueous solution containing an unknown concentration of acetyl salicylate ions was subjected to the above reation procedure, and its acetyl salicylate ion concentration then read from the standard acetyl salicylate calibration curve.

CLAIMS

1. A method for the estimation of a salicylate or a reduced pyridine nucleotide (especially NADH or NAD(P)H) in a sample comprising enzymically converting a salicylate to a catechol in the presence of a reduced pyridine nucleotide and estimating the quantityof catechol obtained, the quantity of catechol being a measure of the salicylate or the reduced pyridine nucleotide in the sample characterised in that the salicylate is converted to the catechol by the action of a salicylate mono-oxygenase enzyme on the salicylate and further characterised in that the quantity of catechol is estimated by reacting the catechol with an aminophenol to form an indophenol and estimating the quantity of indophenol spectrophotometrically.

2. A method according to claim 1 characterised in that the aminophenol comprises para-aminophenol.

3. A method according to claim 2 characterised in that the spectrophotometric estimation of the quantity of indophenol is carried out at a wavelength of 565 nm.

4. A method according to any one of claims 1 to 3 characterised in that the catechol is converted to the indophenol in a solution of pH 12 to 13.

5. A method for the estimation of a salicylate according to any one of claims 1 to 4 characterised in that the salicylate is produced by the enzymic deacetylation of an acetylsalicylate in the presence of an arylester hydrolase enzyme.

6. A method for the estimation of a reduced pyridine nucleotide according to any one of claims 1 to 4 characterised in that the reduced pyridine nucleotide is produced by the enzymic reduction of a pyridine nucleotide in the presence of a lactate and a lactate dehydrogenase enzyme.

7. A method for the estimation of a reduced pyridine nucleotide according to any one of claims 1 to 4 characterised in that the reduced pyridine nucleotide is produced by enzymically converting glucose to glucose-6-phosphate in the presence of ATP and a hexokinase enzyme and subsequently enzymically reducing a pyridine nucleotide in the presence of the glucose-6-phosphate and a glucose-6-phosphate dehydrogenase enzyme.

8. A diagnostic kit for use in the estimation of a salicylate or a reduced pyridine nucleotide (especially NADH or NAD(P)H)in a sample by a method according to claim 1 comprising

a) a salicylate mono-oxygenase enzyme suitable for the conversion of a salicylate to a catechol in the presence of the reduced pyridine nucleotide, and

b) an aminophenol suitable for the conversion of the catechol to an indophenol.

9. A kit according to claim 8 characterised in that the aminophenol comprises para-aminophenol